# DEMANDE DE BREVET EUROPEEN

(11) **EP 1 169 982 A1**
(43) Date de publication de la demande: **09.01.2002**
(21) Numéro de dépôt: 01401768.5
(22) Date de dépôt: 03.07.2001
(51) Int. Cl.: A61F 2/68

(54) **Prothèse active modulaire pour bras et avant-bras**

(30) Priorité: 04.07.2000 FR 0008657
(71) Demandeur: Artigue, Francis, 91140 Villebon-Sur-Yvette (FR)
(72) Inventeur: Artigue, Francis, 91140 Villebon-Sur-Yvette (FR)
(74) Mandataire: Eidelsberg, Olivier

(57) **Abrégé**

Prothèse, notamment du bras et/ou de l'avant-bras, comportant une plaque 1 **;** 11 **;** 21 de fixation destinée à être fixée, notamment par ancrage, à un membre du corps de la personne handicapée adjacent au membre du corps qu'elle est destinée à remplacer, et une barre formant armature 2 ; 12 **;** 22 s'étendant à partir de la plaque 1 ; 11 **;** 21 et d'une longueur correspondant sensiblement à la longueur du membre à remplacer, l'armature 2 ; 12; 22 étant montée articulée sur la plaque de fixation de manière à avoir au moins un degré de liberté et de préférence deux degrés de liberté, par rapport à cette plaque, il est prévu un vérin 4 et de préférence deux vérins 13, 14 **;** 23, 24, montés sensiblement parallèlement à l'axe longitudinal de l'armature.

## Description

La présente invention concerne des prothèses, notamment pour le bras et/ou l'avant-bras, et notamment des prothèses de ce genre électriques.

On connait déjà dans l'art antérieur des prothèses électriques pour le bras, l'avant-bras ou l'ensemble du bras et de l'avant-bras. Ces prothèses de l'art antérieur sont réalisées sur la base de motoréducteurs permettant de transformer une vitesse de rotation d'un moteur rapide en un mouvement lent du bras. Ces prothèses électriques de l'art antérieur sont particulièrement volumineuses, difficiles à mettre en oeuvre, et peu fiables.

La présente invention vise une prothèse électrique, notamment pour le bras et/ou l'avant-bras, d'une conception nouvelle par rapport à l'art antérieur, qui est plus simple à fabriquer, plus simple à mettre en oeuvre, et d'utilisation plus simple et plus fiable pour la personne handicapée.

En particulier, la prothèse suivant l'invention peut être manipulée, notammant par exemple par la personne handicapée elle-même, pour l'amener dans n'importe quelle position de la course possible de la prothèse, et la bloquer cette position même dans le cas où le moteur est en panne. En particulier, la présente invention vise à surmonter l'inconvénient des prothèses de l'art antérieur qui, en cas de panne du système d'entraînement, peuvent se bloquer dans une position telle qu'il faut ensuite démonter ou "déposer" la prothèse pour avoir accès au système d'entraînement, par exemple un moteur, notamment une position de blocage où le bras est complètement replié empêchant l'accès au moteur pour le redémarrer.

Suivant l'invention, une prothèse est telle que définie à la revendication 1.

Dans la description qui suit, le terme vérin désigne un dispositif pouvant prendre une longueur variable soit sous l'action d'un moteur électrique, soit par l'intervention de la personne handicapée ou d'une tierce personne. Dans la suite, on décrira un vérin comme comportant un cylindre et un plongeur pouvant coulisser téléscopiquement dans le cylindre. Cependant, l'invention ne se limite pas à ce mode de réalisation d'un vérin et tout autre dispositif à longueur variable est considéré comme étant englobé par le terme "vérin".

L'armature, qui ici peut être considérée comme constituant le faux bras, va être commandée en rotation suivant son degré de liberté ou ses deux degrés de liberté, pour permettre par exemple dans le cas du bras, de lever ou baisser le bras sur le côté par rapport à la personne handicapée (suivant le plan dans lequel se trouve son buste) ou dans l'autre direction, c'est-à-dire devant elle (suivant le plan vertical perpendiculaire au plan du buste) ou suivant n'importe quel plan compris entre ces deux plans.

Suivant un perfectionnement de l'invention, le vérin est monté articulé sur la plaque de fixation, suivant une liaison à un ou deux degré(s) de liberté. La prothèse est ainsi encore plus souple d'utilisation avec une liberté de mouvement encore plus grande.

Suivant un mode de réalisation avantageux de l'invention, il est prévu également un dispositif de commande du moteur, le dispositif de commande du moteur étant d'une part relié au moteur et d'autre part à la personne handicapée elle-même.

Suivant un mode de réalisation préféré de l'invention, le dispositif de commande du moteur est constitué d'une ou plusieurs électrodes disposées de manière à transmettre des stimuli d'un muscle de la personne handicapée et d'un circuit interpréteur destiné à transformer les stimuli en signaux de commande du moteur.

Suivant un autre mode de réalisation avantageux, le dispositif de commande est constitué d'une commande manuelle par bouton sur lesquels appuie la personne handicapée pour commander le moteur et donc l'entraînement du ou des vérins.

En prévoyant ainsi un dispositif réversible, on peut allonger ou raccourcir le vérin sous effort réduit. C'est-à-dire lorsque le moteur tombe en panne, il est toujours possible d'actionner manuellement le ou les vérins pour ramener le bras dans une position de repos. Dans les dispositifs de l'art antérieur, cette possibilité n'existait pas, et il était nécessaire de prévoir un dispositif de débrayage de l'actionneur électrique en cas de panne ou de défaut de la batterie. Suivant l'invention, il n'est plus nécessaire de prévoir un tel dispositif de débrayage. En outre, en prévoyant un système de court-circuit du moteur, on permet le blocage de la prothèse une fois la position souhaitée atteinte manuellement.

Suivant un autre perfectionnement de l'invention, le ou les vérins est (sont) fixé(s) à leur autre extrémité à une plaque distale en laissant un jeu au niveau de la liaison permettant le déplacement du vérin dans le sens de la longueur du vérin.

En laissant ainsi ce jeu, qui peut être de quelques millimètres seulement, on permet, en position de repos, c'est-à-dire bras pendant et non actionnement du moteur, au bras ou plutôt à la prothèse du bras ou de l'avant-bras de se balancer en imitant le balancement naturel du bras lorsque l'on marche. Le bras ne parait plus rigide comme cela pouvait être le cas dans les prothèses de l'art antérieur, mais au contraire présente un léger balancement lorsque la personne handicapée marche, ce qui fait encore plus ressembler la prothèse à un véritable bras.

Aux dessins donnés uniquement à titre d'exemple, on décrit plusieurs modes de réalisation de prothèses de bras et d'avant-bras suivant l'invention.

La figure 1 est une vue en perspective d'un premier mode de réalisation d'une prothèse de bras ou d'avant-bras comportant un unique vérin (la prothèse étant alors dite mono-directionnelle).

Les figures 2a, 2b et 2c représentent une prothèse de bras à deux vérins (prothèse dite bidirectionnelle), les vérins étant dans différentes positions représentant les différentes possibilités de mouvement de la prothèse de bras.

Les figures 3a et 3b, représentent deux vues d'une prothèse d'avant-bras.

La figure 4 représente une prothèse du membre supérieur comportant une prothèse de bras et une prothèse d'avant-bras.

La figure 5 représente un schéma synoptique de la commande du moteur ou des moteurs de un ou deux vérins d'une prothèse suivant l'invention.

La figure 6 représente une vue éclatée d'un vérin d'une prothèse suivant l'invention.

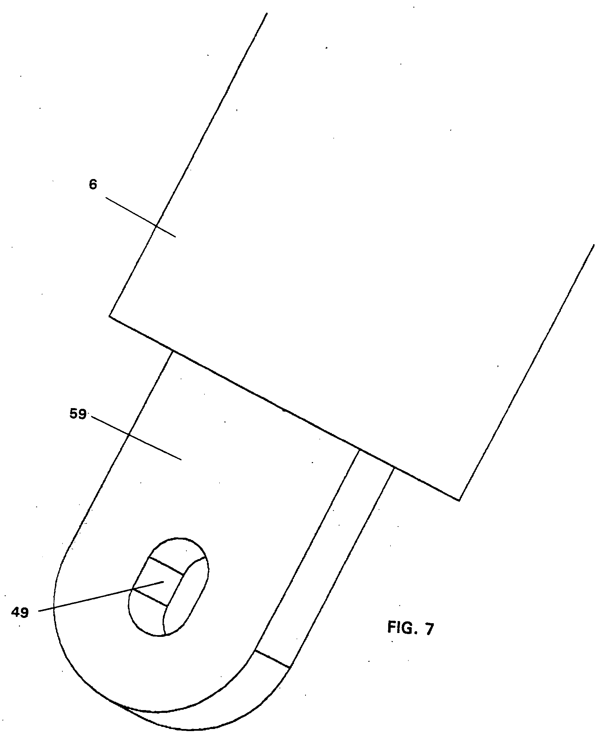

La figure 7 est une vue agrandie d'une partie d'extrémité de la figure 6.

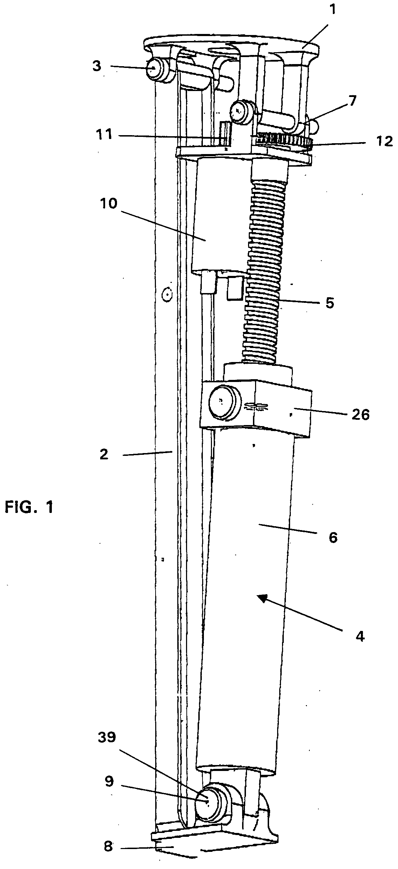

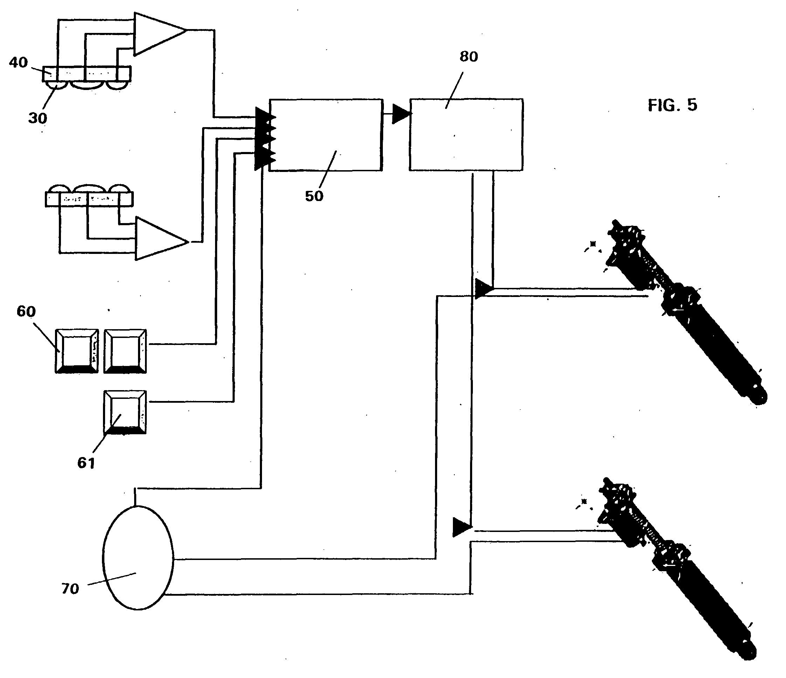

A la figure 1, il est décrit un premier mode de réalisation d'une prothèse, qui peut être aussi bien une prothèse de bras qu'une prothèse d'avant-bras. La prothèse de la figure 1 est constituée d'une plaque 1 de fixation pour la liaison par exemple avec l'épaule dans le cas d'une prothèse de bras ou avec le bras dans le cas d'une prothèse d'avant-bras. Une armature 2 ou barre est fixée à la plaque 1 de fixation par une liaison 3 à charnière simple. L'armature ou barre 2 peut ainsi pivoter suivant un axe de rotation défini par l'axe de la liaison 3. Un vérin 4 est monté sensiblement parallèlement à l'armature 2. Le vérin 4 est constitué d'un plongeur 5 et d'un cylindre 6. Le plongeur 5 du vérin 4 est fixé à son extrémité libre par l'intermédiaire d'une liaison 7 à charnière simple à la plaque 1 de fixation, les axes des deux liaisons 3 et 7 étant parallèles. En outre, la prothèse comporte à son autre extrémité, à savoir l'extrémité distale, une plaque 8 à laquelle est fixée, de manière solidaire en rotation, l'armature 2 et à laquelle est également fixée avec possibilité de rotation suivant une liaison 9 à charnière simple, le cylindre 5 du vérin 4. La prothèse comporte également un moteur électrique 10 qui, par des roues dentées 11 et 12, entraîne la rotation du plongeur 5 et donc son escamotage ou sa sortie du cylindre 6. La roue dentée 11 se trouve à la sortie du moteur en étant entrainée par l'arbre du moteur et la roue dentée 12 engrène dans la roue dentée 11 en étant solidaire en rotation du plongeur 5 de sorte que le moteur, par l'intermédiaire des deux roues dentées, entraîne en rotation le plongeur 5. Ce moteur 10 est commandé par l'intermédiaire d'une part d'électrodes qui transmettent des stimuli d'un muscle ou par l'intermédiaire d'un système de commande à boutons et d'autre part d'un circuit interpréteur. Les systèmes de commande du moteur sont décrits ultérieurement à la figure 5.

Le mode de réalisation de la figure 1 ne comporte qu'un seul vérin. Il permet donc une compréhension simplifiée de la prothèse suivant l'invention. Lorsque le moteur entraîne le plongeur de manière à ce qu'il pénètre plus dans le cylindre 6, l'armature 2 va tourner vers la droite à la figure. De même, lorsque le vérin va être commandé de manière à sortir plus encore du cylindre 6, l'armature 2 va être entraînée dans la direction opposée, c'est-à-dire une rotation vers la gauche. On obtient ainsi sous la commande du moteur 10, la possibilité de faire tourner dans un plan donné, correspondant au plan perpendiculaire à l'axe 3, l'armature 2.

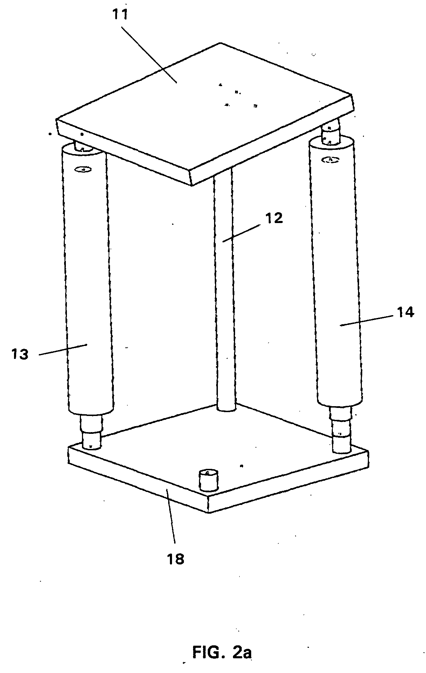

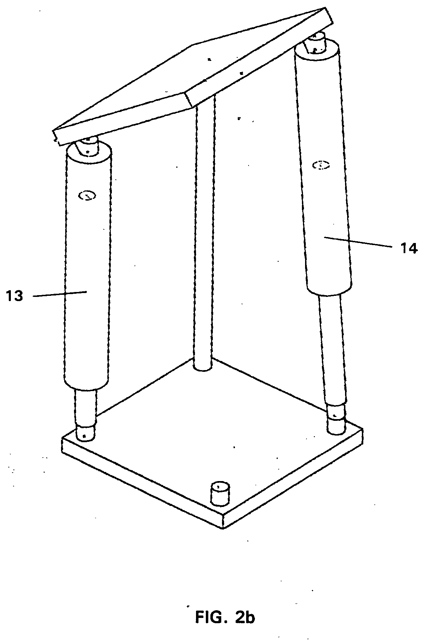

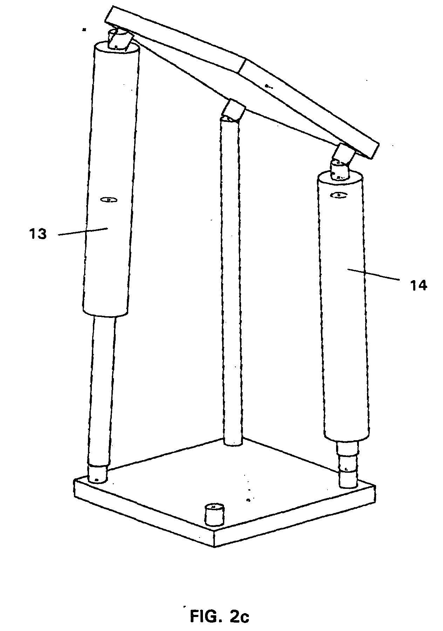

Aux figures 2a, 2b et 2c on a représenté une prothèse de bras comportant cette fois-ci deux vérins. La prothèse de bras des figures 2a, 2b et 2c est constituée d'une plaque 11 destinée à être fixée au niveau de l'épaule à un membre bien portant de la personne handicapée, d'une plaque 18 d'extrémité distale, d'une armature 12 et de deux vérins 13 et 14.

Les deux vérins 13, 14 et l'armature 12 sont fixés aux plaques 11 et 18 suivant cette fois-ci des liaisons cardan, c'est-à-dire permettant une rotation suivant n'importe quel axe parallèle aux plaques. Chaque vérin comporte également un moteur (non représenté) qui sera commandé de la même manière qu'est commandé le moteur 10 de la figure 1.

Lorsque le vérin 13 est actionné par son moteur associé (non représenté) de sorte que le plongeur sorte du cylindre du vérin (figure 2b), l'armature 12 est entraînée en rotation par rapport à la plaque 11 suivant une direction de rotation donnée. Dans le cas où il s'agit d'une prothèse de bras et que la plaque 11 est fixée au niveau de l'épaule, cette rotation correspond par exemple à une rotation de l'armature 12 sur le côté dans le plan du buste de la personne.

Lorsque le vérin 14 est actionné par son moteur associé (non représenté) de sorte que le plongeur sorte du cylindre du vérin (figure 2c) l'armature 12 est entraînée en rotation par rapport à la plaque 11 suivant une direction de rotation perpendiculaire à ladite direction de rotation donnée concernant l'actionnement du vérin 13. Dans le cas où il s'agit d'une prothèse de bras et que la plaque 11 est fixée au niveau de l'épaule, cette rotation correspond à une rotation de l'armature 12 dans le plan vertical perpendiculaire au plan du buste de la personne.

En actionnant simultanément les deux vérins, on peut réaliser une infinité de déplacements de l'armature 12 entre les deux plans définis ci-dessus et on obtient ainsi une prothèse de bras ayant une grande liberté de mouvement.

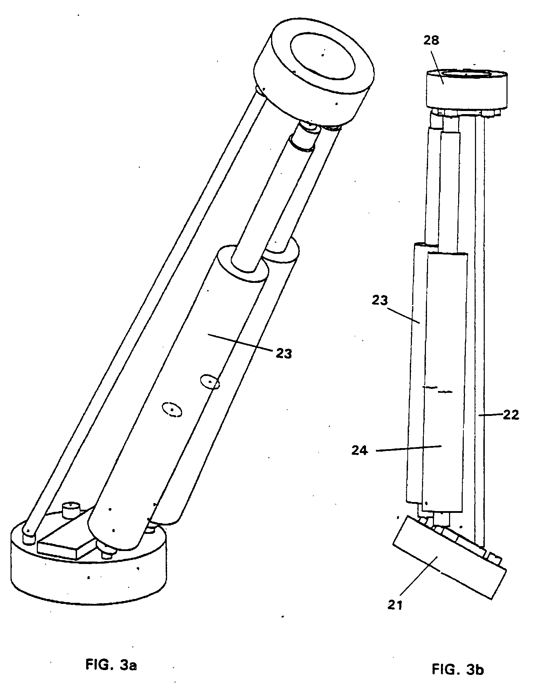

Aux figures 3a et 3b, il est décrit un exemple d'une prothèse d'avant-bras.

Deux vérins 23 et 24 et une barre formant armature 22 sont montés articulés sur deux plaques d'extrémité 21 et 28. L'articulation est une articulation à liaison cardan permettant une rotation de chaque vérin ou de la barre par rapport à n'importe quel axe parallèle aux plaques 21 et 28.

Le fonctionnement de cette prothèse des figures 3a et 3b est rigoureusement identique à ce qui a été décrit pour les prothèses des figures précédentes.

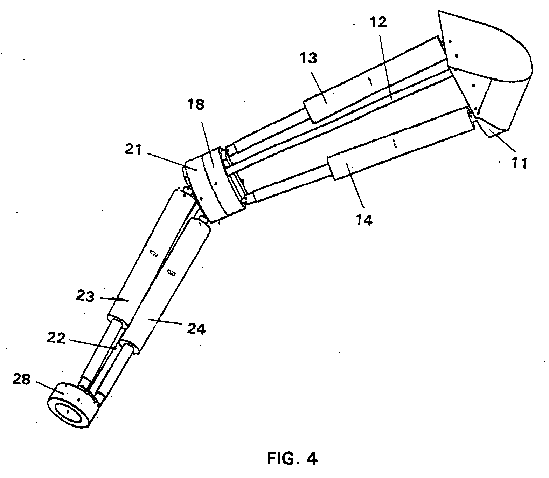

A la figure 4, il est décrit un ensemble d'un membre supérieur constitué d'une prothèse de bras et d'une prothèse d'avant-bras. La plaque distale 18 de la prothèse de bras est fixée à la plaque 21 de fixation de la prothèse d'avant-bras, par exemple par vissage. Les quatre vérins 13, 14, 23 et 24 peuvent être commandés chacun indépendamment des trois autres. On peut également commander le déplacement des vérins simultanément pour permettre ainsi une multitude de déplacements possibles du bras.

La structure des prothèses du bras et de l'avant-bras est identique, la seule différence en étant les dimensions relatives. Suivant cette structure de l'invention, il n'est plus nécessaire d'avoir à disposition deux structures différentes, et donc deux techniques de fabrication différentes, pour le bras et l'avant-bras. Cette modularité simplifie la fabrication, le stockage, l'entretien, l'utilisation, etc.

A la figure 1, on peut voir également que le plongeur 5 du vérin 4 est fileté. En outre, le cylindre 6 du vérin 4 comporte à son extrémité proximale un écrou 26 taraudé dans lequel coulisse le plongeur 5 par coopération de filetage et taraudage. Entre le taraudage de l'écrou 26 et le filetage du plongeur 5, il est prévu des petites billes pour un système d'écrou à billes permettant une réversibilité du vérin. Ainsi, lorsque le moteur tombe en panne ou lorsque les batteries sont usées, il est toujours possible manuellement, grâce au roulement des billes entre le filetage et le taraudage, de faire fonctionner le vérin 4 pour le ramener dans une position de repos. Cette réversibilité permet l'économie de tout dispositif de débrayage du moteur électrique en cas de panne ou de défaut de batterie.

La figure 5 est un schéma décrivant les moyens de commande du moteur entraînant le vérin ou les vérins de la prothèse. Comme on l'a vu précédemment, les vérins sont chacun entraînés par un moteur. Ceux-ci sont alimentés par une source de courant, sous la forme par exemple d'une pile ou d'une batterie. La commande des moteurs, et notamment leur mise en marche et leur fermeture en vu d'entraîner les vérins s'effectue comme suit. Des électrodes 30 sont fixées au niveau de muscles, notamment des muscles 40 rendus inutiles par le handicap. Ces électrodes 30 sont reliées par des fils à un circuit 50 interpréteur. Ce circuit 50 interpréteur, est lui-même relié au circuit de commande du moteur. Lorsque la personne handicapée bande une ou plusieurs fois le ou les muscles 40 auquel est reliée l'électrode 30 ou les électrodes 30, le muscle émet un ou plusieurs stimuli créant un courant de commande transmis par les électrodes au circuit interpréteur. Le circuit 50 interpréteur, en fonction du signal (le ou les stimuli) reçu des électrodes, par exemple le nombre de signaux pendant une période de temps donné, va alors interpréter la commande que souhaite effectuer le patient. Le circuit 50 interpréteur, après avoir ainsi décodé le signal envoyé par la personne handicapée par le bandage de ses muscles, va transmettre des signaux d'instruction à un dispositif 80 de commande du moteur qui va ainsi entraîner le vérin comme le souhaite la personne handicapée. Ainsi, par exemple on peut décider à l'avance et programmer le circuit interpréteur pour que si la personne bande un muscle une fois, c'est qu'elle souhaite, dans le cas par exemple de la prothèse d'un avant-bras, faire tourner son avant-bras vers l'avant, si elle le bande deux fois de suite à un court intervalle de temps, qu'elle souhaite le ramener vers elle, si elle le bande trois fois de suite à un court intervalle de temps qu'elle souhaite le faire tourner vers la droite, et si elle le bande quatre fois de suite à un court intervalle de temps vers la gauche. Ce genre de circuit interpréteur servant à transformer des signaux (stimuli) en des courants actionnant un moteur, et pouvant comporter notamment des transistors et diodes, est bien connu de la technique.

De la même manière, à la place d'un système à électrode, il est également possible de prévoir un système à clavier avec des boutons de commande, la personne handicapée appuyant simplement sur les boutons 60 de commande pour envoyer son signal de commande, un bouton étant prévu par exemple pour tourner la prothèse vers l'avant, un bouton pour la ramener vers elle, un bouton pour la tourner vers la droite ou un bouton pour tourner vers la gauche, etc. Un "joystick." permettant une commande par bâtonnet est également possible.

En outre un capteur 70 de déplacement peut être connecté d'une part au(x) vérin(s) et d'autre part au circuit 50 interpréteur pour prendre en compte la position en cours du bras lors de l'envoi d'instructions au dispositif 80 de commande du moteur.

Enfin on peut également prévoir un bouton 61 interrupteur de verrouillage/déverrouillage qui va permettre de bloquer la prothèse dans la position souhaitée en court circuitant l'alimentation du moteur. Ceci engendre un couple de freinage bloquant la prothèse sans consommation d'énergie. Le court circuit peut être levé par l'interrupteur 61, libérant ainsi les mouvements.

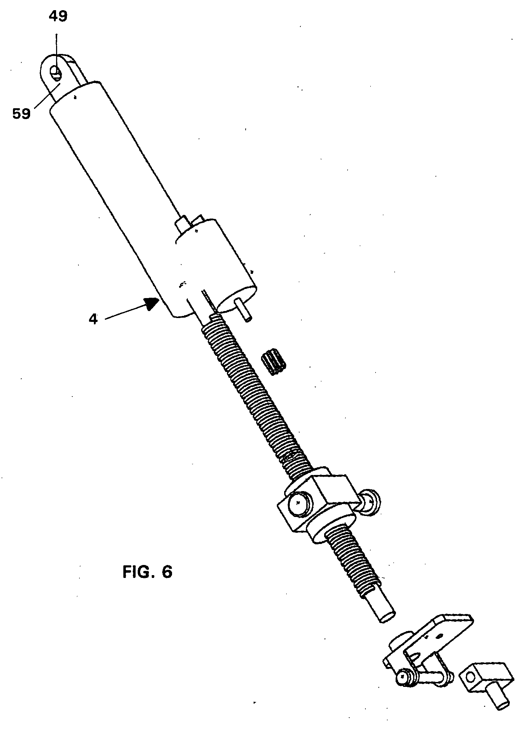

La figure 6 est une vue éclatée du vérin de la figure 1.

Le vérin 4 est constitué d'un cylindre 6 dans lequel peut être escamoté le plongeur 5. La fixation du plongeur 5 à la plaque 1 de fixation s'effectue par l'intermédiaire d'une liaison 7. A l'autre extrémité le cylindre 6 est fixé à la plaque 8, par une liaison 9. Cette liaison 9 est constituée d'un axe 39 cylindrique qui pénètre dans un oeillet 49 formé dans un ergot 59 de prolongement du cylindre 6.

La dimension dans le sens de la longueur du cylindre de l'oeillet 49 est supérieure au diamètre de l'axe 39, ce qui laisse un jeu de un ou plusieurs millimètres (en étant compris notamment entre 0,5 mm et 2,5 mm ici par exemple 1 mm pour un basculement de ± 10°) permettant un léger déplacement longitudinal du vérin. Grâce à ce jeu, l'ensemble de la prothèse va, sans aide du moteur, pouvoir se balancer lorsque la personne handicapée marche, simulant un basculement normal d'un bras pendant la marche.

Dans la description qui précède, on a prévu pour chaque vérin un moteur associé. Cependant, tous les vérins ne sont pas forcément commandés par un moteur et on peut notamment prévoir que certains vérins soient bloqués ou commandés par action directe de la personne handicapée.

## Revendications

1. Prothèse, notamment du bras et/ou de l'avant-bras, comportant une plaque (1 ; 11 ; 21) de fixation destinée à être fixée, notamment par ancrage, à un membre du corps de la personne handicapée adjacent au membre du corps qu'elle est destinée à remplacer ; une armature (2 ; 12 ; 22) s'étendant à partir de la plaque (1 ; 11 ; 21) et d'une longueur correspondant sensiblement à la longueur du membre à remplacer, l'armature (2 ; 12; 22) étant montée articulée sur la plaque de fixation de manière à avoir au moins un degré de liberté par rapport à cette plaque, et au moins un vérin (4) monté sensiblement parallèlement à l'axe longitudinal de l'armature, la prothèse comportant en outre un moteur (10) électrique pour entraîner le déplacement relatif du plongeur (5) du vérin par rapport au cylindre (6) du vérin **caractérisée en ce que** les vérins (4 ; 13, 14 ; 23, 24) sont réalisés suivant un dispositif réversible, notamment le ou les vérins comportent un système à vis-écrou à billes, le plongeur étant fileté pour constituer la vis, le cylindre étant taraudé pour constituer l'écrou et des billes étant insérées dans les filetages entre le plongeur et le cylindre formant écrou, et il est prévu des moyens destinés à court circuiter l'alimentation du moteur.

2. Prothèse suivant la revendication 1, comportant au moins deux vérins, **caractérisée en ce que** les deux vérins définissent chacun avec l'axe de l'armature un plan respectif, les deux plans respectifs n'étant pas parallèles l'un à l'autre, notamment étant perpendiculaires l'un à l'autre.

3. Prothèse suivant la revendication 1 ou 2, **caractérisée en ce que** le ou les vérins est (sont) monté(s) articulé(s) sur la plaque de fixation, suivant une liaison à un ou deux degré(s) de liberté.

4. Prothèse suivant la revendication 1, 2 ou 3, **caractérisé en ce qu'**il est prévu également un dispositif (30, 50, 60) de commande du moteur, dispositif de commande du moteur qui est relié d'une part au moteur et d'autre part à la personne handicapée elle-même.

5. Prothèse suivant la revendication 4, **caractérisée en ce que** le dispositif de commande du moteur est constitué d'une ou plusieurs électrodes (30) disposées de manière à transmettre des stimuli d'un muscle (40) de la personne handicapée et d'un circuit (50) interpréteur destiné à transformer les stimuli en signaux de commande du moteur.

6. Prothèse suivant la revendication 4, **caractérisée en ce que** le dispositif de commande comporte une commande manuelle par boutons (60) sur lesquels appuie la personne handicapée pour commander le moteur et donc l'entraînement du ou des vérins.

7. Dispositif suivant l'une quelconque des revendications précédentes, **caractérisé en ce que** le ou les vérins sont articulés à une plaque (8 ; 18 ; 28) distale suivant une liaison (9) à un degré de liberté ou à deux degrés de liberté, et un jeu est prévu dans la liaison pour permettre un balancement naturel de la prothèse.

8. Prothèse suivant la revendication 7, **caractérisée en ce que** la liaison (9) est réalisée par l'intermédiaire d'un ergot (59) comportant un oeillet (49) et d'un axe (39) cylindrique pénétrant dans l'oeillet (49), l'ergot étant issu du cylindre (6), et la dimension dans le sens de la longueur du cylindre de l'oeillet (49) étant supérieure au diamètre de l'axe (39), notamment en étant compris entre 0,5 mm et 2,5 mm, par exemple 1 mm.

9. Prothèse d'un membre supérieur, comportant une prothèse de bras suivant l'une quelconque des revendications précédentes, fixée à une prothèse d'avant-bras suivant l'une quelconque des revendications précédentes.
